# EUROPÄISCHE PATENTANMELDUNG

(11) **EP 1 314 434 A2**
(43) Veröffentlichungstag der Anmeldung: **28.05.2003**
(21) Anmeldenummer: 01975076.9
(22) Anmeldetag: 22.08.2001
(51) Int. Cl.: A61K 35/78, A61P 3/06

(54) **WIRKSTOFF FÜR DIE REGULIERUNG DES LIPID-METABOLISMUS UND METHODE ZUR HERSTELLUNG DES WIRKSTOFFES**

(30) Priorität: 23.08.2000 RU 2000122087; 12.04.2001 RU 2001109741
(71) Anmelder: Semenov, Sergei Petrovich, St. Petersburg, 193079 (RU)
(72) Erfinder: Semenov, Sergei Petrovich, St. Petersburg, 193079 (RU)
(74) Vertreter: Springstubbe, Wolf, Dipl.-Ing.
(86) Internationale Anmeldenummer: RU0100357
(87) Internationale Veröffentlichungsnummer: WO02015843

(57) **Zusammenfassung**

Die Erfindung ist auf die Schaffung von Mitteln pflanzlicher Herkunft gerichtet, die eine therapeutisch-prophylaktische Wirkung auf den lebenden Organismus ausüben und zur Prophylaxe der Atherosklerose, der Reduzierung des Körpergewichts und zur Senkung der Cholesterinwerte im Blutserum bestimmt sind.

Das Mittel zur Regulierung des Lipidstoffwechsels ist ein durch Behandlung von zerkleinerten Hirsekörnern oder nichtzerkleinerten Haferkörnern mit einem nichtpolaren Extraktionsmittel gewonnener Extrakt.

Das aus Hirsekörnern gewonnene Mittel umfasst eine Lipidfraktion mit einer Dichte von 0,917 bis 0,925 g/cm³ bei 20°C, einer Säurezahl von 120,0 bis 145,0 mg KOH/g, einer Jodzahl von 87,0 bis 93,0 und mehr als 80% freie Fettsäuren.

Das aus Haferkörnern gewonnene Mittel umfasst eine Lipidfraktion mit einer Dichte von 0,760 bis 0,925 g/cm³ bei 20°C, einer Säurezahl von 103,0 bis 110,0 mg KOH/g, einer Jodzahl von 132,0 bis 140, wobei die Lipidfraktion 57 bis 62% freie Fettsäuren und 27 bis 32% und 25 bis 29% Ester der freien Fettsäuren enthält.

Das Verfahren zur Gewinnung eines den Lipidstoffwechsel regulierendes Mittels zeichnet sich dadurch aus, dass zerkleinerte Hirsekörner oder nichtzerkleinerte Haferkörner mit einem nichtpolaren, aus der Gruppe Petrolether, Hexan, Diethylether, Chloroform gewählten Extraktionsmittel bei einem Verhältnis Rohstoff zu Extraktionsmittel von 1:2 bis 1:4 und bei einer Temperatur von 40 bis 70°C behandelt werden mit nachfolgendem Abscheiden und Konzentrieren des Zielproduktes.

Das aus Hirsekörnern oder Haferkörnern gewonnene Mittel erweitert die Auswahl an Mitteln, die für eine therapeutisch-prophylaktische Einwirkung auf den lebenden Organismus vorgesehen sind und eine antisklerotische Wirkung haben.

## Beschreibung

### GEBIET DER TECHNIK.

Die Erfindung betrifft das Gebiet der Medizin, insbesondere die Entwicklung von Mitteln pflanzlicher Herkunft, die eine therapeutisch-prophylaktische Wirkung auf den lebenden Organismus ausüben und zur Prophylaxe der Atherosklerose und zur Senkung des Cholesterinspiegels im Blutserum bestimmt sind.

### BISHERIGER STAND DER TECHNIK

Es sind verschiedene Mittel bekannt, die als Quellen essentieller Fettsäuren für den Menschen lebenswichtig sind.

Einen besonderen Rang nehmen dabei pflanzliche öle ein, zum Beispiel Sonnenblumen-, Mais-, Raps-, Senföl, die Linolsäure enthalten, was sich, den Cholesterinspiegel, den arteriellen Blutdruck u.s.w. senkend, positiv bei der Atherosklerose auswirkt.

Es ist ein Verfahren zur Gewinnung eines Heilproduktes aus Getreide bekannt, umfassend das Einweichen des Rohstoffes im Wasser, Keimen des Getreides, Trocknen und Zerkleinern des getrockneten Getreides (RU-Patent Nr. 2137400, A23L 1/30).

Ein Arzneimittel, das eine prolongierte Wirkung der biologisch aktiven Stoffe aufweist, wird aus Extrakten von durch Adsorption an Getreidekörnern immobilisierten Heilpflanzen gewonnen (RU-Patent Nr. 2111765, A23D 9/00, 1998).
Die Extrakte der Heilpflanzen werden in ein Gefäß gegossen, das Getreidekörner im Verhältnis 1:0,5 enthält. Das Immobilisieren wird innerhalb von 24 bis 36 Stunden durchgeführt, danach werden die Getreidekörner 3 bis 5 Stunden bei einer Temperatur von 55 bis 60°C getrocknet.

Leinöl, das Linolen-, Linol-, Olein- und gesättigte Fettsäuren im Verhältnis 6 bis 8 : 0,5 bis 3 : 0,6 bis 3 : 0,4 bis 3 enthält, wirkt besonders aktiv auf den menschlichen Organismus und übt eine heilende Wirkung aus (RU-Patent Nr. 2158514, A23D 9/00, 2000).

Ein bekanntes Verfahren zur Herstellung eines Heil/Prophylaxeproduktes aus pflanzlichen und/oder tierischen Rohstoffen umfasst dessen Aufbereitung, Extrahieren, Zubereiten eines Extraktes der biologisch wirksamen Stoffe und Vermischen der Rezepturingredienzen zur Zubereitung einer Zusammensetzung mit nachfolgendem Eindampfen bis zu einer Feuchtigkeit von nicht mehr als 22%.
Als pflanzliche Rohstoffe werden Heilpflanzen und/oder Mischungen davon benutzt und als Süßmacher Honig (RU-Patent Nr. 2137400, A23L 1/30, 1/305, 1/308).

Bekannt ist ein Verfahren zur Gewinnung eines Heilproduktes aus Weizen, umfassend das Einweichen des Rohstoffes in Wasser, Keimen des Getreides, Trocknen und Zerkleinern des getrockneten Getreides bis zu einer Korngröße von 20 bis 30 µm (RU-Patent Nr. 2137400, A23L 1/30, 1/305, 1/308).

### OFFENBARUNG DER ERFINDUNG

Die Erfindung ist auf die Schaffung eines den Lipidstoffwechsel des Organismus regulierenden Mittels sowie auf die Entwicklung eines Verfahrens zu seiner Gewinnung gerichtet.

Das erreichte technische Ergebnis liegt in der Erweiterung der Auswahl an Mitteln, die eine therapeutisch-prophylaktische Wirkung auf den lebenden Organismus ausüben und eine antisklerotische Wirkung aufweisen.

Das Mittel zur Regulierung des Lipidstoffwechsels ist ein Extrakt, der durch Behandlung von zerkleinerten Hirsekornern oder nichtzerkleinerten Haferkörnern mit einem nichtpolaren Extraktionsmittel gewonnen wird.

Das aus Hirsekörnern gewonnene Mittel umfasst eine Lipidfraktion mit einer Dichte von 0,917 bis 0,925 g/cm³ bei einer Säurezahl von 120,0 bis 145,0 mg KOH/g, einer Jodzahl von 87,0 bis 93,0 und enthält mehr als 80% freie Fettsäuren.

Das aus Haferkörnern gewonnene Mittel umfasst eine Lipidfraktion mit einer Dichte von 0,760 bis 0,925 g/cm³ bei 20°C, einer Säurezahl von 103,0. bis 110,0 mg KOH/g, einer Jodzahl von 132,0 bis 140, wobei die Lipidfraktion 57 bis 62% freie Fettsäuren und 27 bis 32% und 25 bis 29% Ester der freien Fettsäuren enthält.

Das Verfahren zur Gewinnung eines den Lipidstoffwechsel regulierenden Mittels zeichnet sich dadurch aus, dass zerkleinerte Hirsekörner oder nichtzerkleinerte Haferkörner mit einem nichtpolaren Extraktionsmittel bei deren Verhältnis von 1:2 bis 1:4 und bei einer Temperatur von 40 bis 70°C behandelt werden mit nachfolgendem Abscheiden und Konzentrieren des Zielproduktes.

Als nichtpolares Extraktionsmittel wird ein aus der Gruppe Petrolether, Hexan, Diethylether, Chloroform gewähltes Lösungsmittel verwendet.

Die Extraktion von Hirsekörnern wird im Laufe von nicht weniger als 3 Stunden durchgeführt und die Extraktion von Haferkörnern im Laufe von 6 bis 12 Stunden; das Zielprodukt wird durch Filtrieren abgetrennt, wobei das Zielprödukt durch Eindampfen konzentriert wird.

Die Hirsekörner werden auf eine Teilchengröße von 0,2 bis 0,5 mm zerkleinert.
Der gewonnene Extrakt der zerkleinerten Hirsekörner stellt eine Lipidfraktion der Hirsekörner dar, die sich kennzeichnet durch ihre chemische Zusammensetzung aus mehr als 80% freien Fettsäuren und weniger als 20% Triglyzeriden.
Die chromatographische Analyse der aus den Hafer- und Hirsekörnern gewonnenen Lipidfraktionen wurde an einem einen Gaschromatograph GC-17A, ein Massenspektrometer QP5000 und ein Datenbearbeitungssystem umfassenden Gerätekomplex der Firma Shimadzu (Japan) durchgeführt.
Die Kapillarsäule besteht aus Schmelzquarz SPB (Supeico) mit einer Länge von 25 m und einem Innendurchmesser von 0,2 mm. Die Justierung des Massenspektrometers wurde im automatischen Betrieb mit Perfluortributylamin durchgeführt.
Die Dosierung einer Probe (0,2 µl) wurde im split-Betrieb durchgeführt. Die Temperatur der Säule wurde programmiert von 40°(5 Minuten), bis 270°(20 Minuten) mit einer Geschwindigkeit von 10 Grad/min.

Chromatographische Untersuchungen haben ergeben, dass das aus Hirsekörnern gewonnene Produkt folgende, in Tabelle 1 angegebene chemische Zusammensetzung hat.

**Tabelle 1.**

| Nr. | Bezeichnung der Verbindung | Gehalt in der Probe |
|---|---|---|
| 1 | Linolsäure | 60-70 |
| 2 | Palmitinsäure | 5-7 |
| 3 | Methylester der Linolsäure | 2-4 |
| 4 | Methylester der Octadecansäure | 1,5-3 |
| 5 | Ungesättigte Kohlenwasserstoffe | 3,5-4,4 |
| 6 | Ungesättigte sauerstoffhaltige Aldehyde und Verbindungen der Fettsäuren | Rest |

Das aus Hafer gewonnene Produkt hat folgende, in Tabelle 2 angegebene chemische Zusammensetzung.

**Tabelle 2.**

| Nr. | Bezeichnung der Verbindung | Gehalt in der Probe |
|---|---|---|
| 1 | (9,12-Octadecadien-)Linolsäure | 37-40 |
| 2 | Linolsäure, Methylester | 22-25 |
| 3 | (Hexadecan-)Palmitinsäure | 20-22 |
| 4 | Isopropylpalmitat | 5-7 |
| 5 | Oktadezenalkohol | 4 |
| 6 | Fettalkohol | 0,8 |
| 7 | (Tetradean-)Myristinsäure | 0,4 |
| 8 | Ethylmyristat | 0,1 |
| 9 | Isopropylmyristat | 0,03 |
| 10 | Nichtidentifizierte Alkene | Rest |

Das gewonnene Mittel ist vorgesehen zur Prophylaxe der Atherosklerose und zur Reduzierung des Körpergewichts mittels Stimulierung der fettspaltenden Wirkung des Blutserums, was von einer Senkung der Konzentration von Triglyzeriden und Cholesterin darin begleitet wird.

Untersuchungen haben gezeigt, dass die Verwendung des Mittels nicht nur die Aktivität der Lipase im Organismus stimuliert, sondern gleichzeitig auch Bedingungen zur verstärkten Oxidierung von Fettsäuren mit ihrer überwiegenden Verwendung als Energieversorgungsquelle schafft.

### Beispiel 1.

Hirsekörner mit einer Feuchtigkeit von 12,8% werden bis auf eine Teilchengröße von 0,2 bis 0,5 mm zerkleinert und mittels Petrolether bei einer Temperatur 60°C im Verhältnis Rohstoff : Extraktionsmittel von 1:4 extrahiert. Das Extrahieren wurde in einem Soxhlet durchgeführt, die Extraktionszeit betrug 6 Stunden. Der gewonnene Extrakt wird filtriert und eingedampft bis zur vollen Entfernung des Extraktionsmittels.
Der gewonnene Extrakt stellt eine Lipidfraktion der Hirsekörner dar.
Die Ausbeute an Lipidfraktion aus absolut trockenem Rohstoff betrug 2,4%.

### Eigenschaften des Extrakts:

Farbe: orangegelb
Durchsichtigkeit durchsichtig
Dichte bei 20°C: 0,917 g/cm³.
Brechungsindex bei 20°C: 1,467
Säurezahl, mg KOH pro 1 g: 137,0
Jodzahl (nach Kaufmann): 89,0.

Im Experiment wurden männliche Ratten der Linie Vistar (Rappolovo) mit einer Körpermasse von 190 bis 210 g benutzt. Die Tiere haben die Lipidfraktion von Hirsekörnern in einer Dosis von 0,3 ml 12-mal täglich, 5-mal-in der Woche mittels einer Magensonde erhalten. Nach Ende der Zeitspanne der Verabreichung des Mittels wurden die Ratten dekapitiert, das Blut gesammelt, und nach Bildung eines Gerinnsels wurde das Blutserum durch Zentrifugieren abgetrennt. Im erhaltenen Serum wurde der Gehalt an freien Fettsäuren anhand der Reaktion der Bildung eines Kupferkomplexes ermittelt, der eine Färbung mit Diethylcarbamat ergibt, deren Intensität mit einem Spektralfotometer SF-46 bei einer Wellenlänge von 440 nm gemessen wurde. Die Aktivität der Serumlipase wurde in einer turbidimetrischen Methode geschätzt. Der Gehalt an Triglyzeriden und an Cholesterin gesamt wurde in einem Analysator nach einer enzymatischen Methode ermittelt. Eine Phänotypisierung der Serumlipide wurde anhand einer Analyse ihrer Fraktionszusammensetzung durchgeführt.

Angaben über biochemische Serumkennwerte der Ratten finden sich in Tabelle 3.

**Tabelle 3.**

| Bezeichnung des Kennwertes | Kontrollwert (ohne Verabreichung des Mittels) | Versuchskennwert (12-fache Verabreichung) |
|---|---|---|
| Aktivität der Lipase, % | 13,2 ± 1,3 | 27,8 ± 2,0 |
| Cholesterin, mmol,% | 1,7 ± 0,1 | 1,2 ± 0,1 |
| Triglyzeride, mmol/l | 0,96 ± 0,1 | 0,67 ± 0,08 |
| Lipoproteide hoher Dichte, mmol/l | 0,65 ± 0,08 | 0,56 ± 0,09 |
| Lipoproteide niedriger Dichte, mmol/l | 0,54 ± 0,07 | 0,61 ± 0,1 |
| Lipoproteide sehr niedriger Dichte, mmol/l | 0,46 ± 0,02 | 0,29 ± 0,01 |
| Koeffizient der Atherogenität | 1,53 ± 0,1 | 1,55 ± 0,1 |
| freie Fettsäure, µmol/l | 291,6 ± 32,5 | 273,12 ± 25,2 |

Wie aus den angegebenen Daten hervorgeht, stieg die Aktivität der Lipase des Blutserums bei Verwendung der Lipidfraktion der Hirsekörner auf mehr als das Zweifache. Der Gehalt an Cholesterin insgesamt und an Triglyzeriden sank bei diesen Tieren im Durchschnitt auf 70% bzw. 69,7%. Die Verabreichung des Mittels führte nicht zu einer Änderung des Atherogenitäts-Koeffizienten und des Gehalts der Lipoproteide verschiedener Klassen. Der Gehalt an Lipoproteiden sehr niedriger Dichte verringerte sich auf 63%.

Die erhaltenen Daten zeigen, dass die Verwendung der Lipidfraktion der Hirsekörner die fettspaltende Aktivität des Blutserums stimuliert, was von einer Senkung der Cholesterinkonzentration darin begleitet wird. Die Verabreichung des Mittels aktiviert die Lipase und schafft die Voraussetzungen für eine verstärkte Öxidierung von Fettsäuren mit deren überwiegender Verwendung als Energieversorgungsquelle.

Eine längerdauernde Verabreichung des Mittels (24-malig) führte zu einer Senkung der Konzentration von proatherogenen Lipoproteiden niedriger Dichte und zu einer Verringerung des Atherogenitäts-Koeffizienten mit dem Quotienten 1,8. Außerdem sank der Gehalt an freien Fettsäuren von 279,4 auf 253,0 mmol/l. Diese Änderungen weisen überzeugend eine antiatherogene Wirksamkeit des Mittels bei einer relativ langfristigen Anwendung und die Verminderung des Risikos einer Entwicklung einer Atherosklerose nach.

Angaben zur Änderung der Körpermasse der Ratten, die die Lipidfraktion der Hirsekörner erhalten haben, finden sich in Tabelle 4.

**Tabelle 4.**

| Dauer der Verabreichung des Mittels | Kontrollkennwert | | Versuchskennwert | |
|---|---|---|---|---|
| | Vor Beginn des Versuches | Nach Beendigung des Versuches | Vor Beginn des Versuches | Nach Beendigung des Versuches |
| 12-malige Verabreichung | 192 ± 4,1 | 22,4 ± 2,4 | 203,0 ± 5,7 | 214,0 ± 6,4 |
| ZUNAHME | 16,7 | | 5,4 | |
| 24-malige Verabreichung | 212,0 ± 16,8 | 280,0 ± 16,1 | 199,0 ± 6,1 | 229,0 ± 5,7 |
| ZUNAHME | 32,1 1 | | 15,1 | |

Die angeführtenen Daten zeigen, dass eine 12-malige Aufnahme des Mittels die Zunahme der Körpermasse mit dem Faktor 1/3 bremst und die 24-malige Aufnahme im Durchschnitt mit dem Faktor 1/2.

### BEISPIEL 2.

Haferkörner mit einer Feuchtigkeit von 7,0% werden mittels Petrolether bei einer Temperatur von 60°C im Verhältnis Extraktionsmittel : Rohstoff von 4:1 extrahiert. Das Extrahieren wurde in einem Soxhlet durchgeführt, die Extraktionszeit betrug 6 Stunden. Der gewonnene Extrakt wird gefiltert und eingedampft bis zur vollen Entfernung des Extraktionsmittels.
Der gewonnene Extrakt stellt eine Lipidfraktion der Haferkörner dar.

### Eigenschaften des Extrakts:

Farbe: oliv
Durchsichtigkeit: durchsichtig
Dichte bei 20°C: 0,925 g/cm³
Brechungsindex bei 20°C; 1,475
Säurezahl, (mg KOH pro 1 g): 103,0
Jodzahl: 109
Verseifungszahl: 171.

Es wurde die Einwirkung der Lipidfraktion auf die Kennwerte des Metabolismuszustandes von Lipiden bei Versuchstieren untersucht.

Im Experiment wurden männliche Ratten der Linie Vistar (Rappolovo) mit einer Körpermasse von 160 bis 200 g benutzt. Die Tiere haben die Lipidfraktion in einer Dosis von 0,3 ml 12-mal täglich, 5-mal pro Woche mittels einer Magensonde erhalten. Nach Ende des Zeitraums der Verabreichung der Lipidfraktion wurden die Ratten dekapitiert, das Blut gesammelt, und nach Bildung eines Gerinnsels wurde das Blutserum durch Zentrifugieren abgetrennt. Im erhaltenen Serum wurde der Gehalt an freien Fettsäuren anhand der Reaktion der Bildung eines Kupferkomplexes anhand der Intensität der Färbung mit Diethylcarbamat ermittelt.

Die Aktivität der Serumlipase wurde nach einer turbidimetrischen Methode ermittelt.

Der Gehalt an Triglyzeriden und an Cholesterin gesamt wurde in einem Analysator Spectrom (ABBOT, USA) mittels enzymatischer Methoden ermittelt. Eine Phänotypisierung der Serumlipide wurde anhand einer Analyse ihrer Fraktionszusammensetzung durchgeführt.

Wie die Daten der biochemischen und morphologischen Untersuchungen zeigen, hat die Lipidfraktion keine schädigende Wirkung auf das Parenchym der inneren Organe und zeichnet sich durch eine niedrige allergene Aktivität aus.

Angaben über biochemische Serumkennwerte der Ratten finden sich in Tabelle 5.

**Tabelle 5.**

| Kennwert | Kontrolle | Versuch |
|---|---|---|
| Lipase, Einheiten/l | 12,0 ± 4,3 | 20,3 ± 3,8 |
| Cholesterin, mmol/l | 2,2 ± 0,1 | 2,1 ± 0,2 |
| Triglyzeride, mrnol/l | 0,99 ±0,32 | 1,0 ± 0,24 |
| Lipoproteide hoher Dichte, mmol/l | 0,86 ± 0,11 | 1,25 ± 0,30 |
| Lipoproteide niedriger Dichte, mmol/l | 0,91 ± 0,21 | 0,40 ± 0,15 |
| Lipoproteide sehr niedriger Dichte, mmol/l | 0,42 ± 0,16 | 0,45 ± 0,11 |
| Koeffizient der Atherogenität | 1,55 ± 0,25 | 0,76 ± 0,14 |
| Verhältnis Cholesterin, gesamt/LPHD | 2,78 ± 0,45 | 1,67 ± 0,21 |
| freie Fettsäuren, µmol/l | 312,4 ± 38,6 | 289,6 ± 32,1 |

Wie aus den angegebenen Daten hervorgeht, stieg die Aktivität der Lipase des Blutserums bei den Ratten, die die Lipidfraktion erhalten haben, um 60%. Der Gehalt an Lipoproteiden hoher Dichte stieg um 45%, und der Gehalt an Lipoproteiden niedriger Dichte sank im Durchschnitt um 56%.

Im Ergebnis der Verabreichung der Lipidfraktion sanken die Höhe des Koeffizienten der Atherogenität und das Verhältnis des Gehalts an Cholesterin gesamt des Serums zum Gehalt an Lipoproteiden hoher Dichte auf die Hälfte bzw. um 40%.

Die Lipidfraktion hat ein Antiatherogenpotential, das sich in einer Erhöhung des Gehalts an ein Antiatherogenpotential aufweisenden Lipoproteiden hoher Dichte im Blutserum und in einer Senkung des Gehalts an proatherogenen Lipoproteiden niedriger Dichte bemerkbar macht.

Dabei ist die Höhe des Koeffizienten der Atherogenität im Verhältnis des Gesamtgehalts an Cholesterin zum Gehalt an Lipoproteiden hoher Dichte wesentlich gesunken.

Die wesentliche Erhöhung der Aktivität der Lipase im Serum wird nicht von einer Erhöhung der Konzentration freier Fettsäuren begleitet.

Dies hängt wahrscheinlich damit zusammen, dass die Lipidfraktion ihre Verwertung unter überwiegender Benutzung als Energieträger stimuliert.

Die Anwendung des Lipidextraktes bewirkt zwar keine Senkung von Cholesterin und Triglyzeriden im Serum, bringt aber wesentliche Änderungen im Phänotyp der Lipoproteide, indem er dessen Verschiebung zugunsten der Antiatherogenese bewirkt.

Somit kann das entwickelte Mittel zur Prophylaxe der Atherosklerose angewandt werden.

Angaben zur Dynamik der Körpermasse von Versuchstieren finden sich in Tabelle 6.

**Tabelle 6.**

| Tiergruppe | Körpermasse in Gramm | |
|---|---|---|
| | Vor Verabreichung | Nach Verabreichung |
| KONTROLLE | 170 ± 6 | 192 ± 9 |
| VERSUCH | 170 ± 6 | 178 ± 7 |

Die angeführten Daten zeigen, dass eine tägliche Verabreichung des Extraktes die Zunahme der Körpermasse im Laufe der Beobachtungszeitraums gebremst hat. Eine dauerhafte Verabreichung des Lipidextraktes führt somit bei Ratten zu keiner Änderung des Gehalts an Hämoglobin und Erythrozyten im Peripherieblut.

Bei einer Untersuchung der Zusammensetzung von Formelementen bei, diesen Tieren wurden im Vergleich mit der Kontrollgruppe keine Störungen der Leukozytarformel festgestellt. Somit hat eine dauerhafte Verabreichung des Mittels keine schädigende Wirkung auf das blutbildende System der Tiere.

Das Mittel zeichnet sich durch eine niedrige Allergenwirkung aus.

### GEWERBLICHE VERWERTBARKEIT

Das gewonnene Mittel aus Hirsekörnern oder Haferkörnern erweitert die Auswahl an Mitteln, die für eine therapeutisch-prophylaktische Einwirkung auf den lebenden Organismus vorgesehen sind und eine antisklerotische Wirkung aufweisen.

Die Untersuchungen haben gezeigt, däss Lipidfraktionen aus Hafer- oder Hirsekörnern zur Prophylaxe der Arteriosklerose und zur Reduzierung des Körpergewichts benutzt werden können.

## Patentansprüche

1. Mittel zur Regulierung des Lipidstoffwechsels, sich dadurch auszeichnend, dass es ein durch Behandlung von zerkleinerten Hirsekörnern oder nichtzerkleinerten Haferkörnern mit einem nichtpolaren Extraktionsmittel gewonnener Extrakt ist, wobei
das Mittel aus Hirsekörnern eine Lipidfraktion mit einer Dichte von 0,760 bis 0,925 g/cm³ bei 20°C, einer Säurezahl von 120,0 bis 145,0 mg KOH/g, einer Jodzahl von 87,0 bis 93,0 umfasst und mehr als 80% freie Fettsäuren enthält, und
das Mittel aus Haferkörnern eine Lipidfraktion mit einer Dichte von 0,760 bis 0,925 g/cm³ bei 20°C, einer Säurezahl von 103,0 mg KOH/g, einer Jodzahl von 109 umfasst, wobei die Lipidfraktion 57 bis 62% freie Fettsäuren und 25 bis 29% Ester der freien Fettsäuren enthält.

2. Mittel nach Anspruch 1,
**dadurch gekennzeichnet,**
**dass** es zur Prophylaxe der Atherosklerose vorgesehen ist.

3. Mittel nach Anspruch 1,
**dadurch gekennzeichnet,**
**dass** es zur Reduzierung des Körpergewichts vorgesehen ist.

4. Verfahren zur Gewinnung eines den Lipidstoffwechsel regulierenden Mittels, sich dadurch auszeichnend, dass zerkleinerte Hirsekörner oder nichtzerkleinerte Haferkörner mit einem nichtpolaren Extraktionsmittel bei einem Verhältnis Rohstoff zu Extraktionsmittel von 1:2 bis 1:4 und bei einer Temperatur von 40 bis 70°C behandelt werden mit nachfolgendem Abscheiden und Konzentrieren des Zielproduktes.

5. Verfahren nach Anspruch 4,
**dadurch gekennzeichnet,**
**dass** als nichtpolares Extraktionsmittel ein aus der Gruppe Petrolether, Hexan, Diethylether, Chloroform gewähltes Lösungsmittel verwendet wird.

6. Verfahren nach Anspruch 4,
**dadurch gekennzeichnet,**
**dass** die Hirsekörner auf eine Teilchengröße von 0,2 bis 0,5 mm zerkleinert werden.

7. Verfahren nach Anspruch 4,
**dadurch gekennzeichnet,**
**dass** die Extraktion von Hirsekörnern im Laufe von nicht weniger als 3 Stunden durchgeführt wird.

8. Verfahren nach Anspruch 4,
**dadurch gekennzeichnet,**
**dass** die Extraktion von Haferkörnern im Laufe von 6 bis 12 Stunden durchgeführt wird.

9. Verfahren nach Anspruch 4,
**dadurch gekennzeichnet,**
**dass** das Zielprodukt durch Filtrieren abgetrennt wird.

10. Verfahren nach Anspruch 4,
**dadurch gekennzeichnet,**
**dass** das.Zielprodukt durch Eindampfen konzentriert wird.
